# EUROPEAN PATENT SPECIFICATION

(11) **EP 2 974 680 B1**
(45) Date of publication and mention of the grant of the patent: **10.04.2019**
(21) Application number: 14765189.7
(22) Date of filing: 21.02.2014
(51) Int. Cl.: A61B 17/86, A61B 17/88, A61B 17/92

(54) **MEDICAL SCREW**
MEDIZINISCHE SCHRAUBE
VIS MÉDICALE

(30) Priority: 11.03.2013 JP 2013048387
(43) Date of publication of application: 20.01.2016
(73) Proprietor: Tama Medical Co. Ltd., Hussa-shi, Tokyo 197-0023 (JP)
(72) Inventor: MACHIDA Eiichi, Hussa-shi Tokyo 197-0023 (JP)
(74) Representative: Goddard, Frances Anna
(86) International application number: PCT/JP2014/054167
(87) International publication number: WO 2014/141857

(56) References cited:
- WO-A1-2006/124987
- JP-A- H10 272 142
- JP-A- 2002 143 176
- JP-A- 2003 532 482
- JP-A- 2011 139 901
- US-A- 5 116 337
- US-A1- 2004 015 172
- US-A1- 2007 014 649
- US-A1- 2010 152 740

## Description

### Technical Field

The present invention relates to medical screws for joining bone fractures and jigs for extracting the medical screws.

### Background Art

US 2004/0015172 discloses a bone screw having a tubular thread section, having a bone thread on an outer wall, and an inner thread on an inner wall and extending over the entire length of the thread section. Patent Literature 1 discloses a hollow medical screw 1 called a cannulated screw as illustrated in Fig. 13. The screw 1 has been used for a medical purpose to join bones. The screw 1 has a screw thread 1b on the outer surface of a distal end portion 1a and a drill edge 1c at the tip of the screw 1. There is also formed a through hole 1d along the central axis extending over the entire length of the screw 1. The screw 1 has a head 1e having a recess with a hexagonal cross-section, for example, at the inlet of the through hole 1d to facilitate rotation of the screw 1. The distal end portion of a rotational jig, such as a screwdriver, is fit into the recess 1f and is then rotated. This rotational operation drills the screw thread 1b into a bone fractured region, so that the bone fractures are joined.

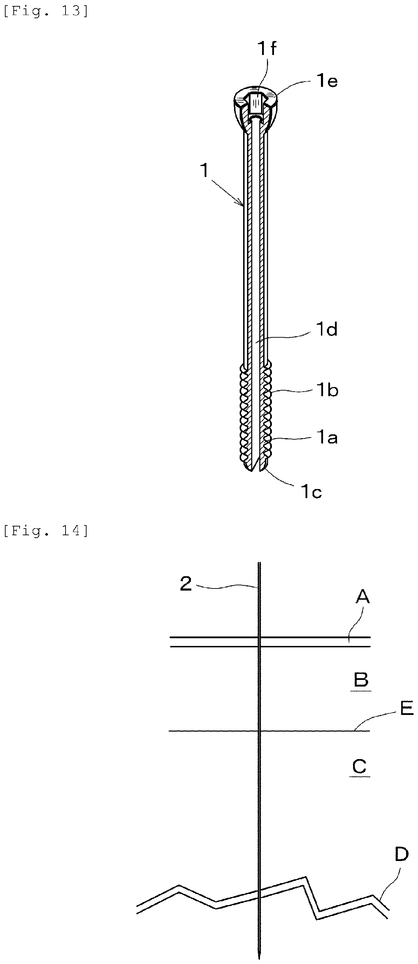

Referring now to Fig. 14, a process of drilling the conventional screw 1 into bone fractures is described: under anesthesia, a metal guide pin 2 is inserted substantially perpendicular to a bone fractured region D of a bone C after a skin A and a subcutaneous tissue B are incised. The guide pin 2 having a sharp drill tip is inserted through the skin A and the subcutaneous tissue B. The guide pin 2 is then rotated until contacting with a bone cortex surface E of the bone C, and is successively drilled into the bone C with a motor, for example.

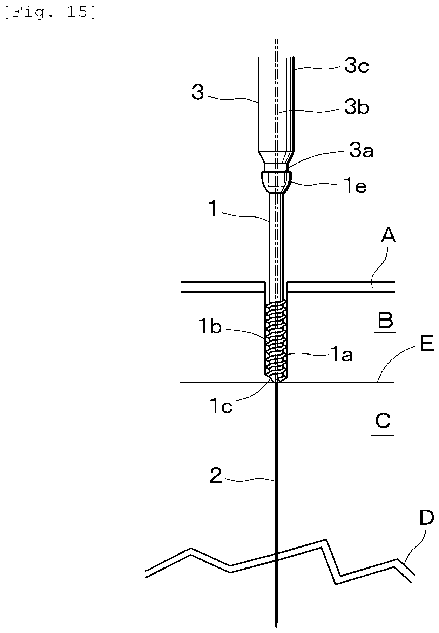

The outlet of the through hole 1d at the distal end portion 1a of the screw 1 is aligned with the proximal end portion of the guide pin 2, and the screw 1 is pushed downward along the guide pin 2. As illustrated in Fig. 15, the proximal end portion of the guide pin 2 projecting upward through the head 1e of the screw 1 is inserted into a through hole 3b of a rotational jig 3. The rotational jig 3 pushes the screw 1 into the skin A and the subcutaneous tissue B. The rotational jig 3 is then manually rotated clockwise while being held at a grip 3c, after distal end portion 1a of the screw 1 contacts with the bone cortex surface E. This clockwise rotational operation of the screw 1 causes the drill edge 1c to dig into the bone C, so that the screw thread 1b is drilled into the bone C along the guide pin 2. Alternatively, the screw 1 may be rotated by the rotational jig 3 such as an electric medical drill, for example.

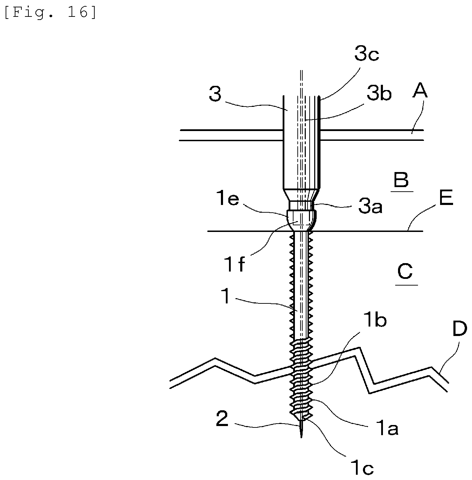

Under observation of an X-ray image on an X-ray TV monitor, the screw 1 is further drilled into the bone C, so that the screw thread 1b penetrates through the bone fractured region D to join bone fractures. As illustrated in Fig. 16, the head 1e of the screw 1 comes into contact with the bone cortex surface E, so that the bone fractured region D is fixed by the screw 1.

At the end of the process, the rotational jig 3 is detached from the guide pin 2, and the guide pin 2 is removed from the bone C. The skin A is sutured to complete the fixing of the bone fractured region D by the screw 1.

After fusion of the bone fractures in the region D in several months, the screw 1 is extracted. After incisions of the skin A and the subcutaneous tissue B, the distal end portion 3a of the rotational jig 3 is fit into the recess 1f of the screw 1 and the rotational jig 3 is rotated counterclockwise. This counterclockwise rotational operation loosens to detach the screw thread 1b from the bone C. An optional guide pin may be inserted in order to guide the rotational jig 3 to the head 1e of the screw 1.

### Citation List

### Patent Literature

[PTL 1] Japanese Unexamined Patent Application Publication No. H10-272142

### Summary of Invention

### Technical Problem

When the bone fractures are fused in the region fixed with the screw 1, a new callus is often generated in the vicinity of the head 1e of the screw 1. The head 1e of the screw 1 is sometimes buried under the callus. At extraction of the screw 1, the callus hinders the rotational jig 3 from rotating counterclockwise; the extraction of the screw 1 thus requires a high rotational force in the counterclockwise direction.

The screw thread 1b of the screw 1 detaches from the bone C in response to a high counterclockwise rotational force applied to the rotational jig 3. During the detaching, the screw thread 1b drilled into the bone C is brought upward to the bone cortex surface E by the counterclockwise rotational force; however, if the head 1e of the screw 1 is still buried in the subcutaneous tissue B, the counterclockwise rotational force slips the screw 1, which hinders the extraction of the screw 1.

In some cases, during the drilling of the screw 1 into the bone C, the screw 1 deviates from the bone C and is drilled into the subcutaneous tissue B. In this case, the screw 1 must be extracted to retry the drilling of the screw 1; however, the counterclockwise rotational operation of the screw 1 slips the screw thread 1b.

In some cases, after several months from the joining operation, the bone C having the drilled screw 1 may cause rejection to metal, generating a gap between the bone C and the screw 1. Also in these cases, the counterclockwise rotational operation of the screw 1 slips the screw thread 1b, which hinders the extraction of the screw 1.

Various types of screws should be prepared which have different lengths or diameters depending, for example, on the position of the bone fractured region D. If a large number of screws 1 having the same length should be used, shortage of the screws 1 may occur. To prevent the problem, a large number of screws 1 having different lengths should be prepared.

A first object of the present invention is to provide a medical screw which solves the above-mentioned problems and can be readily extracted from the bone after fusion of the bone fractures or at replacement of the screw.

A second object of the present invention is to provide a medical screw which is adjustable into a desired length before or during the use.

A third object of the present invention is to provide a removal jig which can securely and readily extract the medical screw.

### Solution to Problem

To achieve the objects described above, the present invention provides a medical screw as defined by independent claim 1. Preferred embodiments are defined by the dependent claims.

### Advantageous Effects of Invention

A medical screw according to the present invention has a screw thread and an inner reverse thread made in the opposite direction to the thread direction of the screw thread. A removal jig to be used with the present invention has an outer reverse thread, which is made in the opposite direction to the thread direction of the screw thread and is formed at a distal end portion of the removal jig. For extracting the medical screw, the outer reverse thread of the removal jig is engaged with an inner reverse thread of the medical screw. The medical screw is thereby integrated with the removal jig. A rotational operation of the removal jig integrated with the medical screw in the opposite direction to the drilled direction of the medical screw facilitates the disengagement and the extraction of the medical screw.

The medical screw may be trimmed (cut) into a desired length depending on a bone fractured region, which eliminates the need for preparation of a large number of medical screws having different lengths.

### Brief Description of Drawings

Fig. 1 is a cross-sectional view of a medical screw according to Embodiment 1.
Fig. 2 is a cross-sectional view of the medical screw held at its proximal end portion by a rotational jig.
Fig. 3 is a cross-sectional view of the medical screw held at its proximal end portion by another rotational jig.
Fig. 4 is a cross-sectional view of the medical screw drilled into a bone fractured region.
Fig. 5 is a schematic view of the medical screw of which proximal end portion is being cut with a cutter.
Fig. 6 is a schematic view of the medical screw having the proximal end portion engaging with a nut.
Fig. 7 is a schematic view of the medical screw and the guide pin inserted into the medical screw at extraction of the medical screw.
Fig. 8 is a schematic view illustrating a removal operation of a callus with a cutting jig.
Fig. 9 is a perspective view of a removal jig.
Fig. 10 is a schematic view of the medical screw and the removal jig engaging with an inner reverse thread of the medical screw.
Fig. 11 is a schematic view illustrating extraction of the medical screw from the bone.
Fig. 12 is a cross-sectional view of a medical screw according to Example 2, which does not fall within the scope of the present invention.
Fig. 13 is a perspective cross-sectional view of a conventional medical screw.
Fig. 14 is a schematic view of a guide pin inserted into the bone fractured region.
Fig. 15 is a schematic view of the conventional medical screw inserted into a subcutaneous tissue.
Fig. 16 is a schematic view of the conventional medical screw drilled into the bone fractured region.

### Description of Embodiments

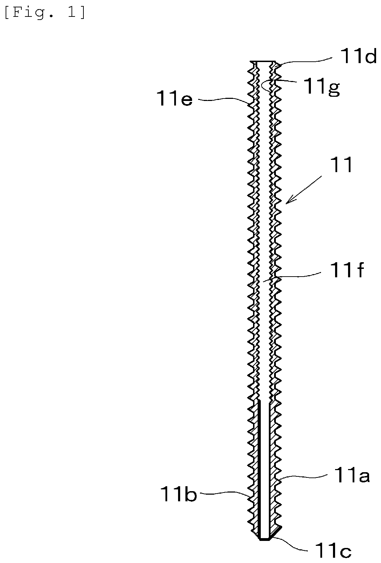

Embodiments of the present invention will now be described in detail with reference to Figs. 1 to 12.

### [Embodiment 1]

Fig. 1 is a cross-sectional view of a medical screw 11 of Embodiment 1. The medical screw 11 has a tubular body composed of a metallic material, such as titanium, a right-hand screw thread 11b extending on the outer surface of a distal end portion 11a of the screw 11, and a drill edge 11c at the tip of the screw 11. A right-hand outer thread 11e extends from a proximal end portion 11d to at least a middle portion on the outer surface of the screw 11. In Embodiment 1, the outer thread 11e is continuous to the screw thread 11b. The screw thread 11b and the outer thread 11e have the same pitch.

The screw 11 has a through hole 11f extending from the distal end portion 11a to the proximal end portion 11d along the central axis of the screw 11. The through hole 11f is configured to receive the metal guide pin 2, which is described in Background Art. The through hole 11f has a circular cross-section having a diameter of approximately 2 mm. A left-hand inner reverse thread 11g, which is made in the opposite direction to the thread direction of the screw thread 11b, extends from the proximal end portion 11d to at least a middle portion on the inner surface of the screw 11. The inner reverse thread 11g may have a different pitch from those of the screw thread 11b and the outer thread 11e.

The screw 11 has an entire length of about 100 mm, for example. It is preferred that various screws having different diameters are prepared and a screw having an appropriate diameter is selected depending on the position and dimension of the bone fractured region D in a phalange or thighbone, for example.

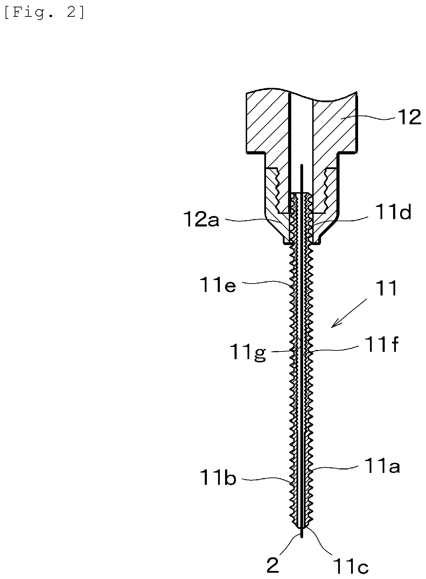

To drill the screw 11 into the bone C, the guide pin 2 is inserted into the bone C after an incision of the skin A and is then inserted into the through hole 11f of the screw 11, under observation of an X-ray image on an X-ray TV monitor, as in the conventional process illustrated in Fig. 14. The proximal end portion 11d of the screw 11 is fixed to the rotational jig, as illustrated in Fig. 2. The proximal end portion 11d of the screw 11 is held with a gripper 12a attached to a chuck 12 of an electric medical drill, for example. If the guide pin 2 hinders the attachment of the chuck 12, the guide pin 2 may be cut into an appropriate length. Alternatively, the chuck 12 may be configured not to hinder an insertion of the guide pin 2 into the through hole 11f.

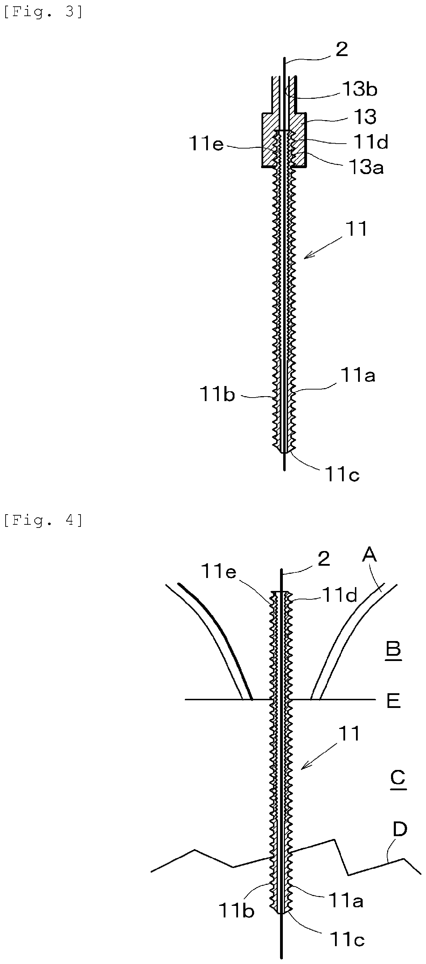

As illustrated in Fig. 3, the rotational jig may be something like a screwdriver having a cap nut 13 at the tip thereof. The cap nut 13 has an inner thread 13a on its inner surface, which is configured to engage with the outer thread 11e. After the engagement of the outer thread 11e with the inner thread 13a, the proximal end portion 11d of the screw 11 which is integrated with the cap nut 13 is rotated, so that the screw 11 is drilled into the bone C. The guide pin 2 can be inserted into a hole 13b of the rotational jig or screwdriver.

Fig. 4 is a cross-sectional view of the screw 11 immediately after the joining of the bone fractures in the region D with the screw 11. The screw 11 is advanced into the subcutaneous tissue B until the distal end portion 11a comes into contact with the bone C. The screw 11 is then rotated clockwise with the rotational jig. This clockwise rotational operation causes the drill edge 11c at the tip of the screw 11 to dig into the bone C, so that the screw thread 11b is drilled into the bone C to join the bone fractures in the region D. After joining the bone fractures in the region D, the rotational jig is detached from the proximal end portion 11d of the screw 11. At this stage, the proximal end portion 11d of the screw 11 projects upward through the bone C.

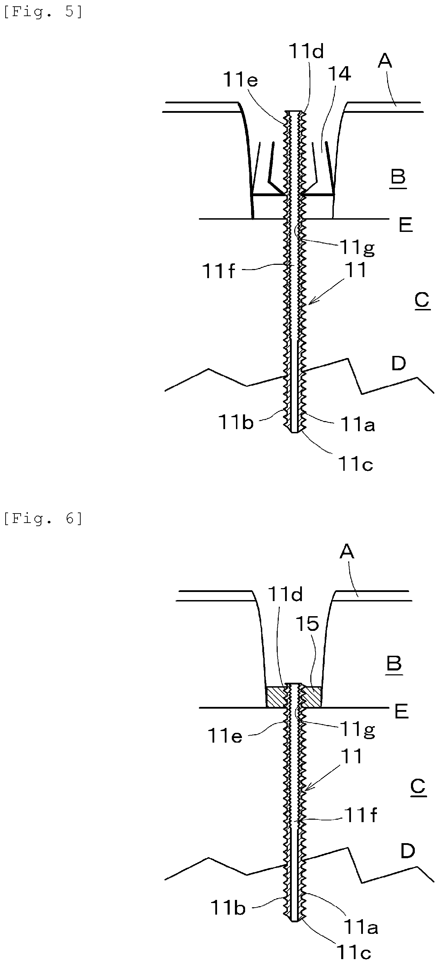

Referring now to Fig. 5, the guide pin 2 is removed and the proximal end portion 11d projecting upward through the bone cortex surface E is cut at its upper end thereof with a cutter 14, such as pliers, into an appropriate length so as to engage with a nut described below. If the rotational jig is an electric drill, the screw 11 and the guide pin 2 may be simultaneously cut with the electrical rotational force of the cutter 14, while the electric drill is being fixed to the screw 11. In this case, the remaining portion of the guide pin 2 in the screw 11 must be removed after the cutting.

Since the outer thread 11e and the inner reverse thread 11g extend from the proximal end portion 11d to at least the middle portion, parts of the outer thread 11e and the inner reverse thread 11g remain on the screw 11 after the cutting at a predetermined position with the cutter 14. The screw 11 should be trimmed without deformation of the cut edge of the screw 11 which may impair the engaging functions of the outer thread 11e and the inner reverse thread 11g. The screw 11 may be trimmed into a required length prior to the drilling of the screw 11.

Referring now to Fig. 6, the remaining part of the outer thread 11e at the proximal end portion 11d of the screw 11 is engaged with a nut 15, and the skin A is sutured for fusion of the bone fractures in the region D. The engagement of the nut 15 prevents displacement of the screw 11 toward the bone C due to rejection occurring with time, for example. The engagement of the nut 15 is not necessarily required if such a risk is not expected.

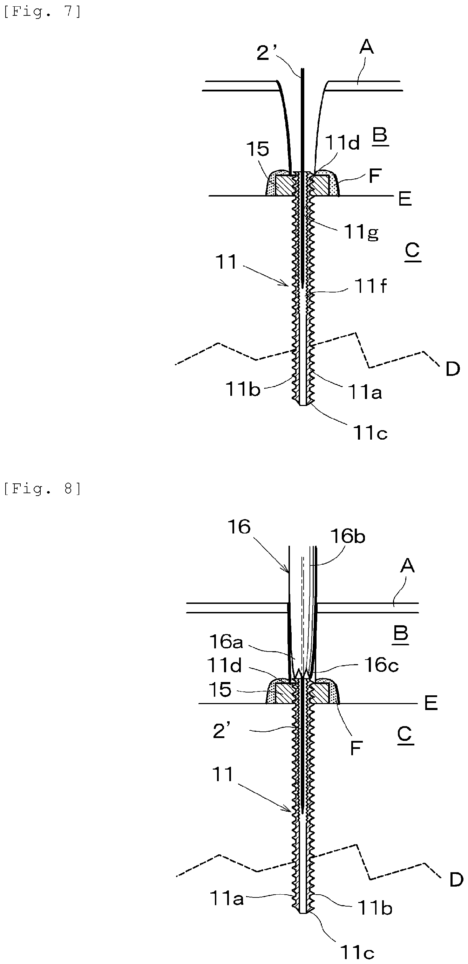

Figs. 7 to 11 illustrates extraction of the screw 11 after fusion of the bone fractures in the region D. At the start of the operation, the skin A is incised under observation of an X-ray TV monitor. As illustrated in Fig. 7, a guide pin 2' is inserted into the through hole 11f from the proximal end portion 11d of the screw 11.

In the extraction of the screw 11, a cutting jig and a removal jig, which are described below, and the screw 11 should pass through the skin A and the subcutaneous tissue B. Taking into account of some expansion and contraction of the skin A and the subcutaneous tissue B, the skin A and the subcutaneous tissue B above the proximal end portion 11d are incised approximately 4 mm in diameter such that the cutting jig and the removal jig can pass through the incisions.

In some cases, a new callus F is generated in the vicinity of the proximal end portion 11d of the screw 11 and the nut 15, and the proximal end portion 11d and the nut 15 are buried under the callus F. In this case, a cutting jig 16 is optionally used to remove the callus F above the nut 15, as illustrated in Fig. 8, so that the inlet of the through hole 11f appears and the nut 15 is removed.

The cutting jig 16 has a cylindrical portion 16b slightly tapered toward a distal end portion 16a so as to readily pass downward through the subcutaneous tissue B. The cutting jig 16 also has a pliable cutting portion 16c for removing the callus F. The cutting portion 16c is disposed at the distal end portion 16a. The cutting portion 16c of the cutting jig 16 has an outer diameter substantially equal to that of the proximal end portion 11d of the screw 11. The diameter of the cutting portion 16c is pliably enlarged in response to pressure on the cutting jig 16.

The upper end of the guide pin 2' is inserted into the hole of the cylindrical portion 16b of the cutting jig 16, and the cutting jig 16 is advanced downward along the guide pin 2' and comes into contact with the callus F generated in the vicinity of the proximal end portion 11d of the screw 11 and the nut 15. The cutting jig 16 is rotated around the guide pin 2' while being pushed against the proximal end portion 11d. This rotational operation causes the cutting portion 16c to cut and remove the callus F above the nut 15. After the removal operation of the callus F, the cutting jig 16 is drawn upward along the guide pin 2'.

The use of the cutting jig 16 is not necessary when the proximal end portion 11d of the screw 11 and the nut 15 are much less buried under the callus F or when the screw 11 is to be replaced with a new one having a different length shortly after the drilling of the screw 11 and before significant generation of a callus F.

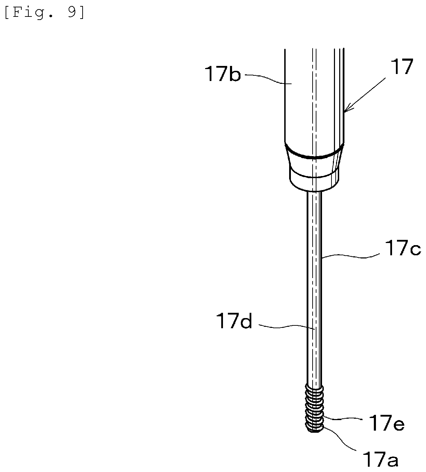

Fig. 9 is a perspective view of a removal jig 17 in the form of a screwdriver to extract the screw 11. The removal jig 17 has a cylindrical insert portion 17c extending from a distal end portion 17a to a grip 17b. An insertion hole 17d having a circular cross-section extends along the central axis of the insert portion 17c. The guide pin 2' can be inserted into the insertion hole 17d.

An outer reverse (i.e., left-hand) screw thread 17e extends on the outer surface of the distal end portion 17a of the insert portion 17c. The outer reverse thread 17e is configured to engage with the inner reverse thread 11g on the inner surface of the screw 11. The outer reverse thread 17e extends over a length of approximately 10 to 20 mm only on the distal end portion 17a so as not to be overscrewed into the through hole 11f of the screw 11.

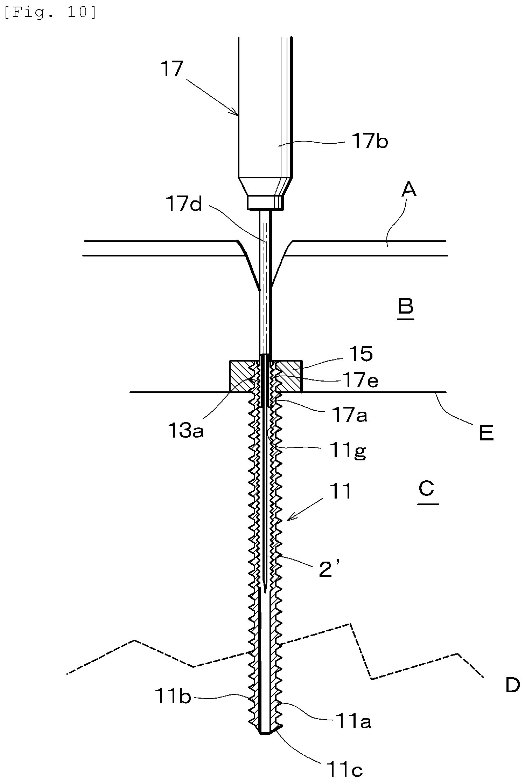

To extract the screw 11, the upper end of the guide pin 2' is inserted into the insertion hole 17d of the removal jig 17, as illustrated in Fig. 10. The removal jig 17 is advanced downward along the guide pin 2', so that the distal end portion 17a of the removal jig 17 is guided into the through hole 11f of the screw 11. The removal jig 17 is then rotated counterclockwise or in a reverse thread direction while being held at a grip 17b. This counterclockwise rotational operation fully engages the outer reverse thread 17e of the removal jig 17 with the inner reverse thread 11g on the inner surface of the screw 11, so that the removal jig 17 is integrated with the screw 11.

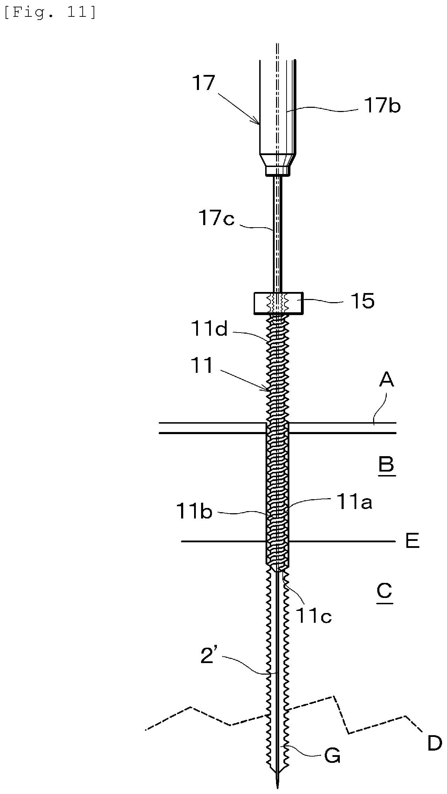

At this stage, the removal jig 17 is further rotated counterclockwise. This counterclockwise rotation is transferred to the screw 11, so that the screw thread 11b of the screw 11 rotates counterclockwise. As illustrated in Fig. 11, the screw begins detaching from the bone C while rotating in the opposite direction to the drilled direction. After the screw 11 is completely detached from the bone C, the counterclockwise rotational operation of the removal jig 17 slips the screw 11; the removal jig 17 integrated with the screw 11 is thus drawn upward to extract the screw 11.

As described above, since the screw 11 is integrated with the removal jig 17, the screw 11 detached from the bone C can be brought upward just by drawing the removal jig 17 upward. The proximal end portion 11d of the screw 11 and the nut 15 are separated from the patient's body, while the incisions of the skin A and the subcutaneous tissue B, which are expanding and contracting, are being widened. At the final stage, the guide pin 2' is removed and the skin A is sutured to complete the extraction. Although the bone C after the extraction of the screw 11 temporarily has a cavity G, the bone C is regenerated over time to fill the cavity G.

The insert portion 17c of the removal jig 17 to be inserted into the skin A has a diameter substantially identical to that of the through hole 11f of the screw 11; a smaller incision of the skin A is required before inserting the removal jig 17 into the skin A to extract the screw 11. A screw 11 with a nut 15 attached to the proximal end portion 11d can be extracted, while the incision of the skin A is being widened.

The screw 11 may be extracted without the guide pin 2' . In this case, under observation of an X-ray TV monitor, the skin A and subcutaneous tissue B above the proximal end portion 11d of the screw 11 are each incised a substantially identical diameter to the outer diameter of the proximal end portion 11d. The removal jig 17 is inserted through the incisions into the subcutaneous tissue B, so that the outer reverse thread 17e is engaged with the inner reverse thread 11g.

In the above description, a single screw 11 is used in one bone fractured region D; however, multiple screws 11 may be used in a large bone fractured region D. An optional perforated metal plate serving as a splint may be disposed over the bone fractured region D of the bone C, and the screws 11 may be inserted through the holes of the metal plate to join the bone fractures in the region D.

### [Embodiment 2]

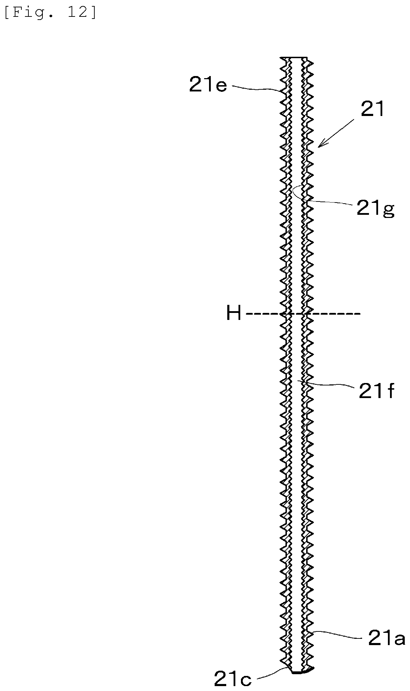

Fig. 12 is a cross-sectional view of a medical screw 21 according to Example 2. The screw 21 has an entire length of at least 100 mm and a through hole 21f. An inner reverse (left-hand) thread 21g extends on the inner surface over the entire length of the screw 21. In addition, an outer (right-hand) thread 21e extends over the entire length of the screw 21.

In the joining process of the bone fractures in the region D, the screw 21 may be cut along Line H with a cutter. The separated upper end portion of the screw 21 may be used as another screw 21 to join bone fractures in another region D. For reuse of the other screw 21, the tip of the distal end portion 21a of the other screw 21 is processed into a drill edge 21c with a grinder, for example, and the outer thread 21e serves as a screw thread.

Alternatively, the screw 21 may be cut at Line H to form another screw 21 having a sharp saw-edged tip. The other screw 21 can be reused without a process with a grinder, for example.

In practical use, an elongated medical screw 21 cannot be stably drilled while being held at its proximal end portion. In this case, the guide pin 2 may be inserted to measure the depth of the bone fractured region D before drilling the medical screw 21, and the screw 21 to be drilled into the bone fractured region D may be trimmed into an appropriate length depending on the measured depth.

The cutting jig 16 and removal jig 17 described in Embodiment 1 can also be used in Example 2.

In Embodiment 1 and Example 2 described above, the medical screws 11 and 21 each have an outer thread in the form of a right-hand thread and an inner thread in the form of a left-hand thread; however, the relation may be vice versa.

### Reference Signs List

- 2, 2': guide pin
- 11, 21: medical screw
- 11a, 21a: distal end portion
- 11b: screw thread
- 11c, 21c: drill edge
- 11d: proximal end portion
- 11e, 21e: outer thread
- 11f, 21f: through hole
- 11g, 21g: inner reverse thread
- 12: chuck
- 13: cap nut
- 14: cutter
- 15: nut
- 16: cutting jig
- 16c: cutting portion
- 17: removal jig
- 17a: distal end portion
- 17c: insert portion
- 17e: outer reverse thread

## Claims

1. A medical screw (11) for joining bone fractures, the medical screw (11) comprising:
a metal tubular body;
a screw thread (11b) extending on the outer surface of a distal end portion (11a) of the tubular body (11),
an outer thread (11e) extending from a proximal end portion (11d) to at least a middle portion on the outer surface of the tubular body (11), the screw thread and the outer thread being made in a first thread direction, the outer thread (11e) being continuous to the screw thread (11b), the screw thread (11b) and the outer thread (11e) having the same pitch;
a through hole (11f) extending from the distal end portion (11a) to the proximal end portion (11d) along the central axis of the tubular body, the through hole (11f) having a constant diameter from the distal end portion to the proximal end portion of the tubular body, the through hole being configured to receive a guide pin (2, 2') thereinto, and
an inner reverse thread (11g) extending from the proximal end portion (11d) to at least a middle portion on the inner surface of the tubular body, the inner reverse thread (11g) being made in a second thread direction opposite to the first thread direction;
**characterised in that** the outer thread (11e) at the proximal end portion is configured to engage with a nut (15).

2. The medical screw (11) according to claim 1, wherein the tubular body is adjustable in length by trimming with a cutter (14).

3. The medical screw (11) according to claim 1 or 2, wherein a metal guide pin (2, 2') is insertable into the through hole (11f).

## Patentansprüche

1. Medizinische Schraube (11) zum Zusammenfügen von Knochenbrüchen, wobei die medizinische Schraube (11) Folgendes umfasst:
einen rohrförmigen Metallkörper; ein Schraubengewinde (11b), das sich an der Außenfläche eines distalen Endabschnitts (11a) des rohrförmigen Körpers (11) erstreckt,
ein Außengewinde (11e), das sich von einem proximalen Endabschnitt (11d) zu mindestens einem Mittelabschnitt der Außenfläche des rohrförmigen Körpers (11) erstreckt,
wobei das Schraubengewinde und das Außengewinde in einer ersten Gewinderichtung hergestellt sind, wobei das Außengewinde (11e) fortlaufend zu dem Schraubengewinde (11b) ist, wobei das Schraubengewinde (11b) und das Außengewinde (11e) die gleiche Steigung aufweisen;
ein Durchgangsloch (llf), das sich von dem distalen Endabschnitt (11a) zu dem proximalen Endabschnitt (11d) entlang der Mittelachse des rohrförmigen Körpers erstreckt, wobei das Durchgangsloch (11f) einen konstanten Durchmesser von dem distalen Endabschnitt zu dem proximalen Endabschnitt des rohrförmigen Körpers aufweist, wobei das Durchgangsloch konfiguriert ist, um einen Führungsstift (2, 2') darin aufzunehmen, und
ein inneres Umkehrgewinde (11g), das sich von dem proximalen Endabschnitt (11d) zu mindestens einem Mittelabschnitt an der Innenfläche des rohrförmigen Körpers erstreckt, wobei das innere Umkehrgewinde (11g) in einer zweiten Gewinderichtung entgegengesetzt zu der ersten Gewinderichtung hergestellt ist; **dadurch gekennzeichnet, dass** das Außengewinde (11e) an dem proximalen Endabschnitt konfiguriert ist, um mit einer Mutter (15) in Eingriff zu treten.

2. Medizinische Schraube (11) nach Anspruch 1, wobei der rohrförmige Körper in der Länge durch Zuschneiden mit einer Schneideeinrichtung (14) einstellbar ist.

3. Medizinische Schraube (11) nach Anspruch 1 oder 2, wobei ein Metallführungsstift (2, 2') in das Durchgangsloch (11f) einführbar ist.

## Revendications

1. Vis médicale (11) pour souder des fractures osseuses, la vis médicale (11) comprenant :
un corps tubulaire en métal ;
un filetage de vis (11b) s'étendant sur la surface extérieure d'une partie d'extrémité distale (11a) du corps tubulaire (11),
un filetage extérieur (11e) s'étendant d'une partie d'extrémité proximale (11d) jusqu'à au moins une partie médiane de la surface extérieure du corps tubulaire (11), le filetage de vis et le filetage extérieur étant réalisés dans une première direction de filetage, le filetage extérieur (11e) étant situé dans le prolongement du filetage de vis (11b), le filetage de vis (11b) et le filetage extérieur (11e) ayant le même pas ;
un trou traversant (11f) s'étendant de la partie d'extrémité distale (11a) jusqu'à la partie d'extrémité proximale (11d) le long de l'axe central du corps tubulaire, le trou traversant (11f) ayant un diamètre constant de la partie d'extrémité distale jusqu'à la partie d'extrémité proximale du corps tubulaire, le trou traversant étant configuré pour recevoir une broche de guidage (2, 2'), et
un filetage inversé intérieur (11g) s'étendant de la partie d'extrémité proximale (11d) jusqu'à au moins une partie médiane de la surface interne du corps tubulaire, le filetage inversé interne (11g) étant réalisé dans une seconde direction de filetage opposée à la première direction de filetage ;
**caractérisé en ce que** le filetage extérieur (11e) au niveau de la partie d'extrémité proximale est configuré pour venir en prise avec un écrou (15).

2. Vis médicale (11) selon la revendication 1, dans laquelle le corps tubulaire est réglable en longueur par découpe à l'aide d'un couteau (14).

3. Vis médicale (11) selon la revendication 1 ou 2, dans laquelle une broche de guidage en métal (2, 2') peut être insérée dans le trou traversant (11f).
